(19) Europäisches Patentamt
European Patent Office
Office européen des brevets

(11) Veröffentlichungsnummer: **0 007 576**
**B1**

(12) **EUROPÄISCHE PATENTSCHRIFT**

(45) Veröffentlichungstag der Patentschrift: **16.11.83**

(21) Anmeldenummer: **79102536.4**

(22) Anmeldetag: **18.07.79**

(51) Int. Cl.³: **C 07 C 69/743,** A 01 N 37/10, A 01 N 37/24, A 01 N 53/00, C 07 C 67/14, C 07 C 69/65, C 07 C 120/00, C 07 C 69/734, C 07 C 69/753, C 07 C 69/747, C 07 C 121/75 //C07C67/313, C07C67/32

(54) **Ester von Stilbenderivaten, Verfahren zu ihrer Herstellung, Stilbenalkohole als Zwischenprodukte, Verwendung der Ester als Insektizide.**

(30) Priorität: **21.07.78 DE 2832213**

(43) Veröffentlichungstag der Anmeldung:
**06.02.80 Patentblatt 80/3**

(45) Bekanntmachung des Hinweises auf die Patenterteilung:
**16.11.83 Patentblatt 83/46**

(84) Benannte Vertragsstaaten:
**BE CH DE FR GB IT NL**

(56) Entgegenhaltungen:
**DE - A - 2 531 456**
**US - A - 3 705 919**

**Journal of the American Chemical Society, Vol. 75 (1953) Seiten 3452-3458**

**Die Akte enthält technische Angaben, die nach dem Eingang der Anmeldung eingereicht wurden und die nicht in dieser Patentschrift enthalten sind.**

(73) Patentinhaber: **BAYER AG**
**Zentralbereich Patente, Marken und Lizenzen**
**D-5090 Leverkusen 1, Bayerwerk (DE)**

(72) Erfinder: **Förster, Heinz, Dr.**
**Am Eckbusch 47**
**D-5600 Wuppertal 1 (DE)**
Erfinder: **Fuchs, Rainer, Dr.**
**Roeberstrasse 8**
**D-5600 Wuppertal 1 (DE)**
Erfinder: **Hammann, Ingeborg, Dr.**
**Belfortstrasse 9**
**D-5000 Köln 1 (DE)**
Erfinder: **Stendel, Wilhelm, Dr.**
**In den Birken 55**
**D-5600 Wuppertal 1 (DE)**

**EP 0 007 576 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Ester von Stilbenderivaten, Verfahren zu ihrer Herstellung, Stilbenalkohole als Zwischenprodukte, Verwendung der Ester als Insektizide

Die Erfindung betrifft neue Stilbenderivate, mehrere Verfahren und Zwischenprodukte zu ihrer Herstellung sowie ihre Verwendung als Insektizide.

Es ist bekannt, daß 2,2-Dichlorvinyloxy-benzylester von bestimmten Cyclopropancarbonsäuren, wie z.B. 2-(2,2-Dichlorovinyl)-3,3-dimethyl-cyclopropan-1-carbonsäure-$\alpha$-cyano-3'-(2,2-dichlorvinyloxy)-benzylester, zur bekämpfung von Insekten verwendet werden können (vergleiche DE—OS 2 554 883).

Die Wirkung dieser Verbindungen ist jedoch, insbesondere bei niedeigen Aufwandmengen, nicht immer zufriedenstellend.

Gegenstand der vorliegenden Erfindung sind

1. neue Stilbenderivate der Formel I

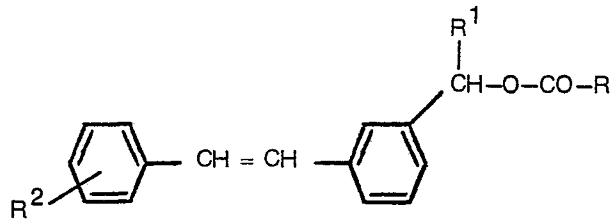

(I)

in welcher

R für einen offenkettigen oder cyclischen Alkylrest steht, der gegebenenfalls substituiert ist durch Halogen, Alkyl, Cycloalkyl, Alkenyl, welches gegebenenfalls durch Halogen substituiert ist, Styryl, welches gegebenenfalls durch Halogen substituiert ist, Phenyl, welches gegebenenfalls durch Halogen, Alkyl, Alkoxy, Alkylthio, Halogenalkyl, Halogenalkoxy, Halogenalkylthio oder Methylendioxy substituiert ist, spirocyclisch verknüpftes Cycloalkenyl, welches gegebenenfalls benzannelliert ist und

$R^1$ für Wasserstoff, Cyan oder Alkinyl steht und

$R^2$ für Wasserstoff, Halogen oder Alkyl steht;

2. ein Verfahren zur Herstellung der neuen Stilbenderivate der Formel I, das dadurch gekennzeichnet ist, daß man Carbonsäuren der Formel II

R—COOH (II)

in welcher

R die oben angegebene Bedeutung hat oder ein Salz oder Säurechlorid derselben mit Alkoholen der Formel III

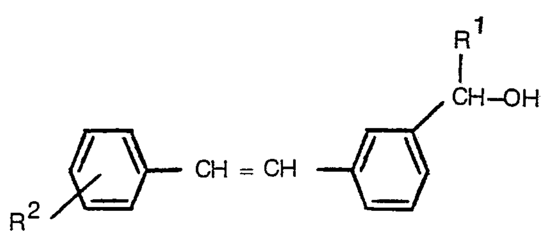

(III)

in welcher

die Reste $R^1$ und $R^2$ die oben angegebene Bedeutung haben,

oder für den Fall, daß $R^1$ für CN steht, die Säurechloride der Säuren der Formel III mit den den Alkoholen der Formel III zugrundeliegenden Aldehyden und Alkalicyaniden, gegebenenfalls in Gegenwart eines Säurebinders und gegebenenfalls in Gegenwart eines Verdünnungsmittels umsetzt.

Als Aldehyde werden Formyl-stilbenderivate der Formel IV

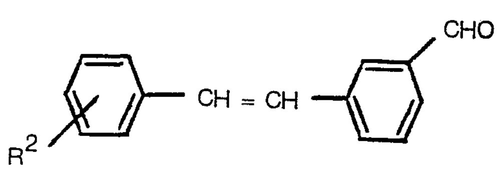

(IV)

in welcher

$R^2$ die oben angegebene Bedeutung hat; verwendet. Diese Verbindungen werden enhalten indem,

man Alkohole der Formel III mit Phosphortribromid umsetzt und die entstehenden Brommethyl-stilbenderivate der Formel X

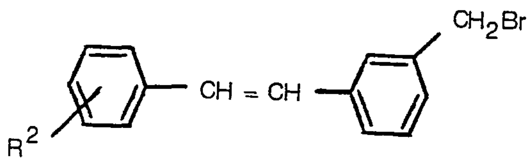

(X)

in welcher
$R^2$ die oben angegebene Bedeutung hat hydrolysiert.

3. Neue Alkohole der Formel III

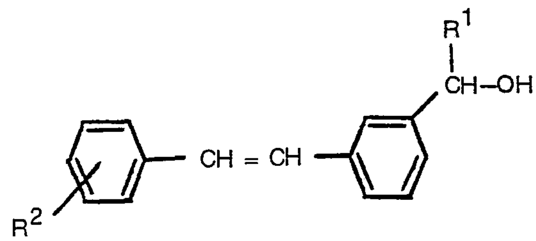

(III)

in welcher
$R^1$ für CN steht und
$R^2$ für Wasserstoff, Halogen oder Alkyl steht.

Die Alkohole der Formel III, gemäß 3 (oben), worin $R^1$ für Wasserstoff steht, werden erhalten, indem man eine Verbindung der Formel V

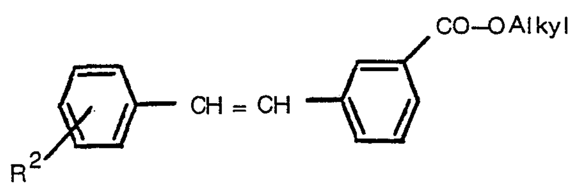

(V)

in welcher
$R^2$ die oben angegebene Bedeutung hat und Alkyl für $C_{1-4}$-Alkyl steht,
mit einem Reduktionsmittel gegebenenfalls in Gegenwart eines Verdünnungsmittels umsetzt.

Verbindungen der Formel V werden erhalten, indem man

a) Phenylessigsäurederivate der Formel VI

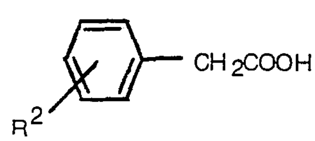

(VI)

in welcher
$R^2$ die oben angegebene Bedeutung hat mit 3-Formylbenzoesäureestern der Formel VII

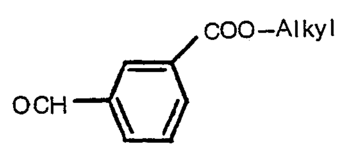

(VII)

umsetzt und die dabei erhaltenen Verbindungen der Formel VIII

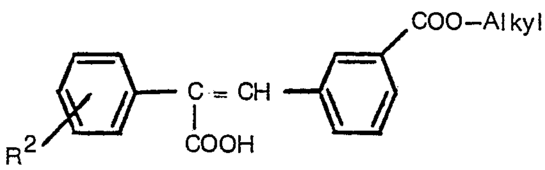

(VIII)

in welcher

$R^2$ und Alkyl die oben angegebene Bedeutung haben decarboxyliert, oder

b) Benzylphosphonsäureester der Formel IX

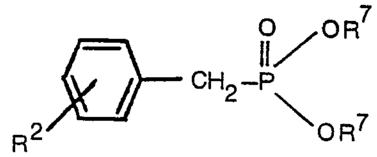

(IX)

in welcher

$R^2$ die oben angegebene Bedeutung hat und

$R^7$ für Alkyl oder Phenyl steht oder beide Reste $R^7$ für Alkandiyl, stehen

mit einer Base und mit einem 3-Formylbenzoesäureester der Formel VII umsetzt oder

c) Benzyltriphenylphosphoniumsalze der Formel IX a

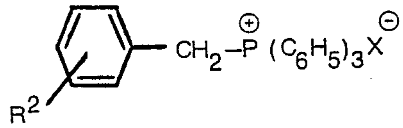

(IX a)

in welcher

$R^2$ die oben angegebene Bedeutung hat und

X für Halogen steht,

mit einer Base und mit einem 3-Formylbenzoesäureester der Formel VII umsetzt;

Bevorzugt sind neue Stilbenderivate der Formel I

in welcher

R für den Rest

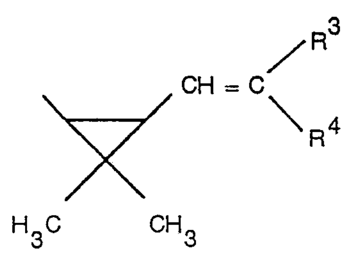

steht, wobei $R^3$ für Wasserstoff, Methyl, Chlor oder Brom und $R^4$ für Methyl, Chlor, Brom oder für gegebenenfalls halogensubstituiertes Phenyl steht oder wobei $R^3$ und $R^4$ zusammen für Alkandiyl stehen, oder in welcher

R für den Rest

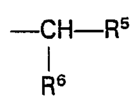

steht, wobei $R^5$ für gegebenenfalls durch Halogen, Alkyl, Alkoxy, Alkylthio, Halogenalkyl, Halogenalkoxy, Halogenalkylthio oder Methylendioxy substituiertes Phenyl steht und $R^6$ für Isopropyl oder Cyclopropyl steht, oder in welcher

R für einen der Reste

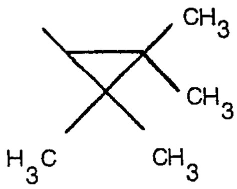 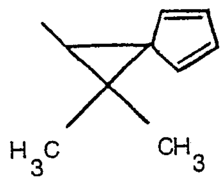 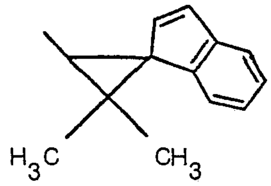 oder 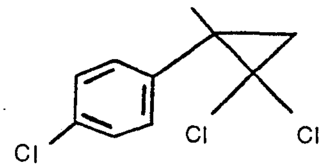

steht, in welcher weiter

$R^1$ für Wasserstoff, Cyano oder Äthinyl steht und

$R^2$ für Wasserstoff, Halogen oder Alkyl steht,

gefunden.

Gegenstand der Erfindung sind insbesondere Stilbenderivate der Formel (I), in welcher

R für den Rest

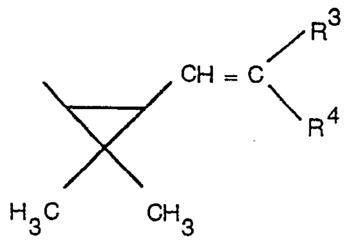

steht, worin

$R^3$ für Wasserstoff, Methyl, Chlor oder Brom und

$R^4$ für Methyl, Chlor, Brom, Phenyl oder Chlorophenyl steht oder worin

$R^3$ und $R^4$ zusammen für Aklandiyl mit 2 bis 6 Kohlenstoffatomen stehen, oder in welcher

R für den Rest

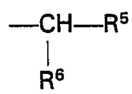

steht, wobei

$R^5$ für Phenyl, Chlorophenyl, Methylphenyl, Methoxyphenyl, Methylthiophenyl, Trifluormethylphenyl, Trifluormethoxyphenyl, Trifluormethylthiophenyl oder 3,4-Methylendioxyphenyl steht und

$R^6$ für Isopropyl oder Cyclopropyl steht, oder in welcher

R für einen Reste

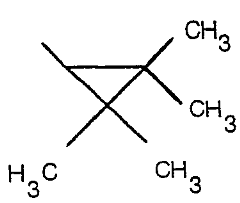 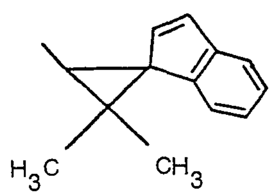 oder 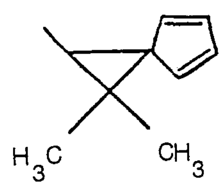

steht, in welcher weiter

$R^1$ für Wasserstoff oder Cyano steht und

$R^2$ für Wasserstoff steht.

In der allgemeinen Formel (I) sind die verschiedenen Isomeren und deren Mischungen mit eingeschlossen.

Die neuen Stilbenderivate der Formel (I) werden bevorzugt erhalten, wenn man als reaktionsfähige Derivate der Carbonsäuren der Formel II Carbonsäurechloride der Formel XI

$$R—CO—Cl \qquad (XI)$$

in welcher

R die oben angegebene Bedeutung hat, mit Alkoholen der Formel III

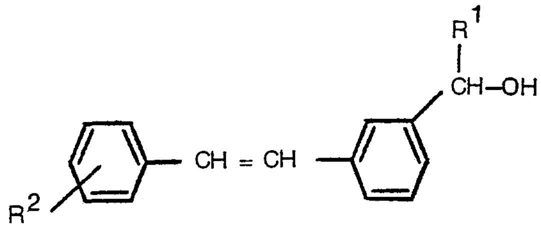

(III)

in welcher die Reste $R^1$ und $R^2$ die oben angegebene Bedeutung haben, gegebenenfalls in Gegenwart eines Säureakzeptors und gegebenenfalls in Gegenwart eines Verdünnungsmittels umsetzt. Ein besonders bevorzugtes Verfahren zur Herstellung von Verbindungen der Formel (I), worin $R^1$ für Cyano steht, ist dadurch gekennzeichnet, daß man Carbonsäurechloride der Formel XI (oben) mit Formylstilben-derivaten der Formel IV

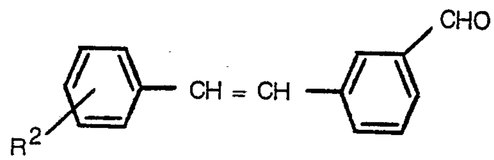

(IV)

in welcher

$R^2$ die oben angegebene Bedeutung hat, in Gegenwart von wenigstens der äquimolaren Menge Alkylicyanid, gegebenenfalls in Gegenwart eines Katalysators und gegebenenfalls in Gagenwart von Verdünnungsmitteln umsetzt.

Überraschenderweise zeigen die neuen Stilbenderivate der Formel I eine bessere insektizide Wirkung als die entsprechenden bekannten Produkte analoger Konstitution und gleicher Wirkungsrichtung. Sie stellen somit eine wertvolle Bereicherung der Technik dar.

Verwendet man beispielsweise zur Herstellung der Stilbenderivate der Formel I 3-(2-(4-Chlorophenyl)-vinyl)-benzyl-alkohol und $\alpha$-Isopropyl-4-chloro-phenyl-essigsäurechlorid oder 3-(2-Phenyl-vinyl)-benzaldehyd und 2,2-Dimethyl-3-(2-chloro-2-phenyl-vinyl)-cyclopropan-1-carbonsäurechlorid sowie Natriumcyanid als Ausgangsstoffe, so können die entsprechenden Reaktionen durch folgende Formelschemata skizziert werden:

(a)

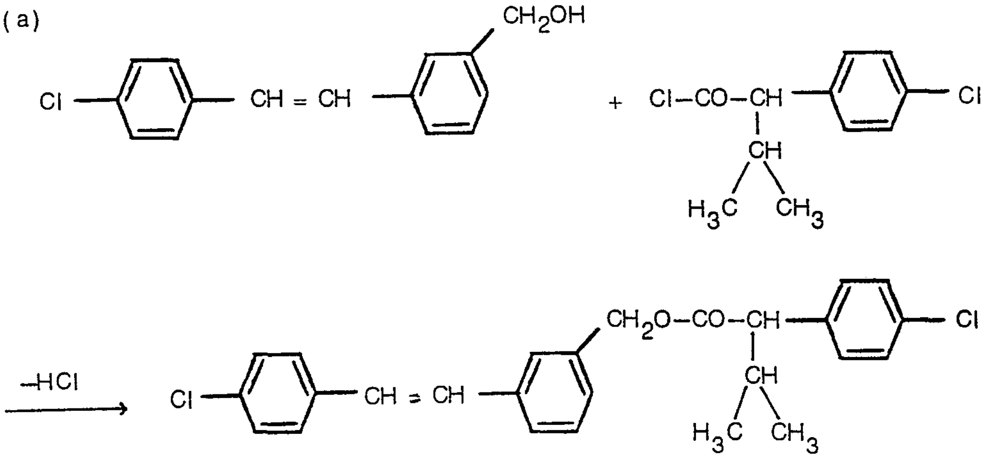


(b)

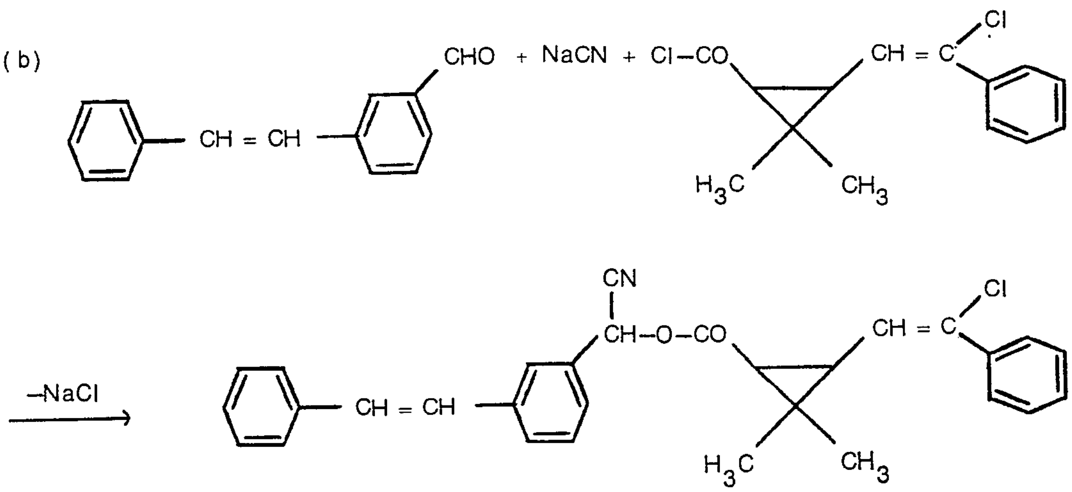


Die zu verwendenden Ausgangsstoffe sind durch die Formeln (II), (III), (IV) und (XI) definiert.

Vorzugsweise stehen darin die Reste R, $R^1$ und $R^2$ für diejenigen Reste, welche bei der Definition der Reste R, $R^1$ und $R^2$ in Formel (I) als bevorzugt genannt wurden.

Die als Ausgangsverbindungen zu verwendenden Carbonsäuren der Formel (II) bzw. ihre reaktionsfähigen Derivate wie Säurechloride oder Ester sind bekannt (vergleiche DE—A 1 926 433, 2 231 312, 2 365 555, 2 605 828 und 2 738 150 sowie 2 544 150):

Als Beispiele für die Carbonsäurechloride der Formel (XI) seien im einzelnen genannt:

2,2-Dimethyl-3-(2-methyl-propen(1)yl)-,
2,2-Dimethyl-3-(2,2-dichloro-vinyl)-,
2,2-Dimethyl-3-(2,2-dibromo-vinyl)-,
2,2-Dimethyl-3-(2-phenyl-vinyl)-,
2,2-Dimethyl-3-(2-(4-chloro-phenyl)-vinyl)-,
2,2-Dimethyl-3-(2-chloro-2-phenyl-vinyl)- und
2,2-Dimethyl-3-(2-chloro-2-(4-chloro-phenyl)-vinyl-cyclopropan-1-carbonsäurechlorid, ferner
α-Isopropyl-phenylessigsäurechlorid,
α-Isopropyl-4-chloro-phenyl-essigsäurechlorid,
α-Isopropyl-4-trifluormethyl-phenyl-essigsäurechlorid,
α-Isopropyl-4-methoxy-phenyl-essigsäurechlorid,
α-Isopropyl-4-trifluormethoxy-phenylessigsäurechlorid,
α-Isopropyl-4-methylthio-phenyl-essigsäurechlorid,
α-Isopropyl-4-trifluormethylthio-phenyl-essigsäurechlorid,
α-Isopropyl-3,4-methylendioxy-phenyl-essigsäurechlorid,
α-Cyclopropyl-phenyl-essigsäurechlorid und
α-Cyclopropyl-4-chloro-phenyl-essigsäurechlorid, ferner
3-Cyclopropyliden-methyl-, 3-Cyclobutyliden-methyl- und
3-Cyclopentyliden-methyl-2,2-dimethyl-cyclopropan-1-carbonsäurechlorid, sowie
2,2,3,3-Tetramethyl-cyclopropan-1-carbonsäurechlorid,

2,2-Dimethyl-spiro(2,4)hepta-4,6-dien-1-carbonsäurechlorid und 3,3-Dimethyl-spiro-(cyclopropan-1,1'-inden-2-carbonsäurechlorid. Als Beispiele für die Carbonsäuren der Formel II seien die den oben erwähnten Säurechloriden der Formel XI zugrundeliegenden genannt.

Die als Ausgangsverbindungen zu verwendenden Alkohole der Formel (III) sind neu. Man erhält Verbindung der Formel (III), worin

$R^2$ die oben angegebene Bedeutung hat und

$R^1$ für Wasserstoff steht, indem man Alkoxycarbonylstilben-derivate der Formel (V)

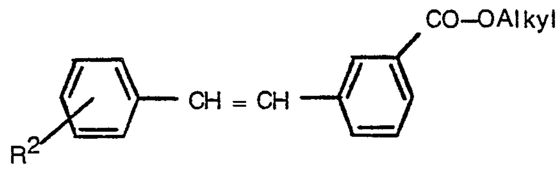

(V)

worin

$R^2$ die oben angegebene Bedeutung hat,

mit einem Reduktionsmittel wie z.B. Lithiumaluminiumhydrid, gegebenenfalls in Gegenwart eines inerten Verdünnungsmittels wie z.B. Diäthyläther, Tetrahydrofuran oder Dioxan, bei Temperaturen zwischen 0 und 50°C umsetzt.

Die Aufarbeitung kann auf übliche Weise erfolgen, indem man beispielsweise mit Wasser verdünnt, mit einer starken Säure wie z.B. Salzsäure ansäuert, mit einem organischen Lösungsmittel wie z.B. Toluol extrahiert und nach Trocknen das Lösungsmittel abdestilliert.

Als Beispiel für die Alkohole der Formel III seien Phenylvinyl- oder Styryl-benzylalkohole, insbesondere 3-(2-Phenylvinyl)-benzylalkohol, genannt.

Die als Zwischenprodukte zu verwendenden Alkoxycarbonylstilbenderivate sind durch Formel (V) definiert. Vorzugsweise stehen darin

$R^2$ für Wasserstoff und

Alkyl für Methyl oder Äthyl.

Als Beispiele seien genannt:

3-(2-Phenyl-vinyl)-benzoesäure-methylester und -äthylester.

Die Alkoxycarbonyl-stilben-derivate der Formel (V). Erhält man indem man

(a) Phenylessigsäure-derivate der Formel

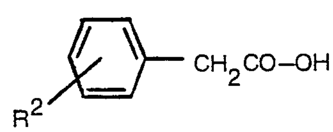

(VI)

worin

$R^2$ die oben angegebene Bedeutung hat, mit 3-Formyl-benzoesäureestern der Formel

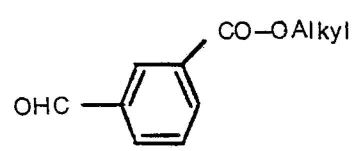

(VII)

gegebenenfalls in Gegenwart eines basischen Katalysators wie z.B. Triäthylamin und gegebenenfalls in Gegenwart eines Verdünnungsmittels wie z.B. Essigsäureanhydrid, bei Temperaturen zwischen 20 und 200°C, vorzugsweise zwischen 50 und 150°C, zu Verbindungen der Formel

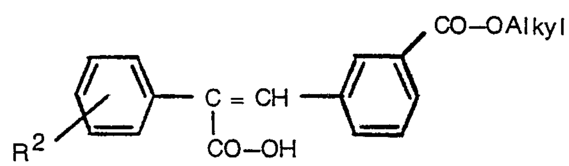

(VIII)

worin

$R^2$ die oben angegebene Bedeutung hat, umsetzt und die Verbindungen der Formel (VIII), gegebenenfalls nach Reinigung durch Umkristallisation, durch Erhitzen auf Temperaturen zwischen 150 und 300°C, vorzugsweise zwischen 200 und 250°C, gegebenenfalls unter Verwendung eines

Verdünnungsmittels wie z.B. Chinolin und gegebenenfalls unter Verwendung eines Katalysators wie z.B. Kupferpulver, decarboxyliert.

Die Aufarbeitung kann hierbei auf übliche Weise erfolgen, z.B. durch Versetzen mit Toluol und Sälzsäure, Abtrennen der organischen Phase, Waschen und Trocknen, Abziehen des Lösungsmittels und Destillation des Rückstandes im Vakuum.

Alkoxycarbonyl-stilben-derivate der Formel (V) erhält man auch, indem man

(b) Benzylphosphonsäureester der Formel

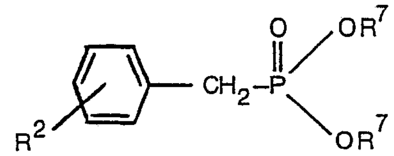

(IX)

worin

$R^2$ die oben angegebene Bedeutung hat und

$R^7$ für Alkyl oder Phenyl steht oder beide Reste $R^7$ für Alkandiyl stehen,

mit einer Base wie z.B. Natriummethylat, gegebenenfalls unter Verwendung eines Verdünnungsmittels wie z.B. Methanol und/oder Tetrahydrofuran und dann mit 3-Formyl-benzoesäureestern der oben stehenden Formel (VII) bei Temperaturen zwischen —50 und +100°C, vorzugsweise bei —10 bis +50°C, umsetzt.

Es kann dann auf übliche Weise aufgearbeitet werden, z.B. durch Aufnehmen der Reaktionsmischung in Toluol, Waschen mit Wasser, Trocknen der organischen Phase, Abziehen des Lösungsmittels und Destillation des Rückstandes im Vakuum sowie gegebenenfalls Umkristallisation.

Alkoxycarbonyl-stilben-derivate der Formel V erhält man ferner auch, indem man

c) Benzyl-triphenyl-phosphoniumsalze der Formel

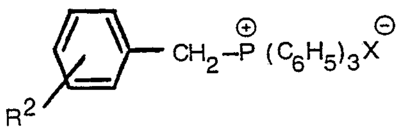

(IX a)

worin

$R^2$ die oben angegebene Bedeutung hat und

X für Halogen steht,

mit einer Base wie z.B. Natriumäthylat gegebenenfalls unter Verwendung eines Verdünnungsmittels wie z.B. Äthanol und dann mit einem 3-Formyl-benzoesäureester der Formel VII (oben), vorzugsweise mit 3-Formylbenzoesäurebutylester, bei Temperaturen zwischen —50 und +100°C, vorzugsweise bei —10 bis +50°C, umsetzt. Die Aufarbeitung kann auf übliche Weise durchgeführt werden, z.B. durch Einengen, Extraktion mit Toluol und Abziehen des Lösungsmittels im Vakuum.

Die nach dem Herstellungsverfahren (a) erhaltenen Alkoxycarbonyl-stilben-derivate liegen im allgemeinen zu über 90% in der cis-Konfiguration vor;

nach Verfahren (b) erhält man dagegen die entsprechenden Produkte zu über 90% in der trans-Konfiguration und nach Verfahren (c) fallen die Isomeren etwa im Verhältnis 1:1 an.

Die als Ausgangstoffe zu verwendenden Phenylessigsäure-derivate sind durch Formel (VI) definiert. Vorzugsweise steht darin

$R^2$ für Wasserstoff.

Als Beispiel sei Phenylessigsäure genannt.

Die Phenylessigsäure-derivate der Formel (VI) sind bekannte Verbindungen.

Die weiter als Ausgangsstoffe zu verwendenden 3-Formyl-benzoesäureester sind durch Formel (VII) definiert. Vorzugsweise steht darin Alkyl für Methyl oder Äthyl.

Als Beispiele seien genannt:

3-Formyl-benzoesäure-methylester und

3-Formyl-benzoesäure-äthylester.

Die 3-Formyl-benzoesäureester der Formel (VII) sind bekannte Verbindungen (vergleiche Ber. Deut. Chem. Ges. *71* (1938), 335—341).

Die ferner als Ausgangsstoffe zu verwendenden Benzylphosphonsäureester sind durch Formel (IX) definiert. Vorzugsweise stehen darin

$R^2$ für Wasserstoff und

$R^7$ für Methyl, Äthyl, Phenyl oder 2,2-Dimethyl-propan(1,3)diyl.

Als Beispiel sei genannt:

Benzyl-phosphonsäure-dimethylester, -diäthylester und -diphenylester.

Die Benzylphosphonsäureester der Formel (IX) sind bekannte Verbindungen.

Die als Ausgangsstoffe verwendbaren Benzyltriphenylphosphoniumsalze sind durch Formel IX a definiert. Vorzugsweise stehen darin

$R^2$ für Wasserstoff und

X für Chlor.

Als Beispiel sei Benzyl-triphenyl-phosphoniumchlorid genannt.

Die Benzyl-triphenyl-phosphoniumsalze der Formel IX a sind bekannt.

Die bei der besonders bevorzugten Verfahrensweise zur Herstellung von Verbindungen der Formel (I), worin $R^1$ für Cyano steht als Ausgangsstoffe zu verwendenden Formyl-stilben-derivate (Phenylvinyl-benzaldehyd-derivate) sind durch Formel (IV) definiert.

Vorzugsweise steht darin

$R^2$ für Wasserstoff.

Als Beispiel für die Verbindungen der Formel (IV) sei 3-(2-Phenyl-vinyl)-benzaldehyd genannt.

Die Formyl-stilben-derivate der Formel (IV) kann Man ausgehend von Hydroxymethyl-stilbenderivaten der Formel (III), worin

$R^2$ die oben angegebene Bedeutung hat und

$R^1$ für Wasserstoff steht,

nach bekannten Verfahren herstellen, z.B., indem man Hydroxymethyl-stilben-derivate der Formel (III) mit Phosphortribromid, gegebenenfalls unter Verwendung eines Verdünnungsmittels, bei Temperaturen zwischen 0 und 50°C umsetzt, die entstehenden Brommethyl-stilben-derivate der Formel

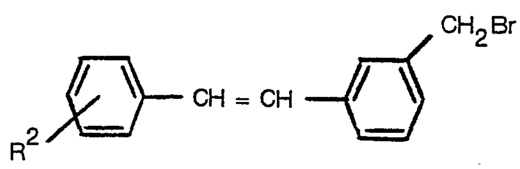

(X)

worin

$R^2$ die oben angegebene Bedeutung hat,

auf übliche Weise, nach Waschen der Reaktionsmischung mit Wasser. Trocknen und Abziehen des Lösungsmittels, isoliert und sie mit Hexamethylentetramin, gegebenenfalls unter Verwendung eines Verdünnungsmittels, bei Temperaturen zwischen 0 und 80°C umsetzt, die kristallinen Addukte mit wässriger Essigsäure auf Temperaturen zwischen 80 und 120°C erhitzt, nach Abkühlen aus der mit Salzsäure versetzten Lösung mit Methylenchlorid extrahiert, trocknet und destilliert.

Die Verfahren zur Herstellung der erfindungsgemäßen Verbindungen der Formel (I) werden im allgemeinen unter Verwendung von Verdünnungsmitteln durchgeführt. Als solche kommen praktisch alle inerten organischen Solventien infrage sowie gegebenenfalls — bei Arbei ten im Zweiphasenmedium — auch Wasser als zweite Solvenskomponente. Als organische Lösungsmittel für die erfindungsgemäßen Verfahren seien genannt: aliphatische und aromatische, gegebenenfalls chlorierte Kohlenwasserstoffe, wie z.B. Pentan, Hexan, Heptan, Benzol, Toluol, Xylol, Methylenchlorid, Chloroform, Tetrachlorkohlenstoff, Chlorbenzol und Dichlorbenzol, Äther wie z.B. Diäthyläther, Tetrahydrofuran und Dioxan sowie Nitrile wie z.B. Acetonitril und Propionitril.

Bei den als bevorzugt erwähnten Verfahren, ausgehend von Säurechloriden der Formel XI und Alkoholen der Formel III, können als Säureakzeptoren alle üblichen Säurebindemittel verwendet werden. Insbesondere seien genannt: Alkalicarbonate wie z.B. Natrium- und Kaliumcarbonat, Alkalialkoholate wie z.B. Natrium- und Kaliummethylat sowie Natrium- und Kaliumäthylat, ferner aliphatische, aromatische oder heterocyclische Amine wie z.B. Trimethyl- und Triäthylamin, Dimethylanilin, Dimethylbenzylamin und Pyridin.

Bei den als besonders bevorzugt erwähnten Verfahren, ausgehend von Formylstilbenderivaten der Formel IV und Säurechloriden der Formel XI, werden als Katalysatoren im allgemeinen Verbindungen verwendet, welche gewöhnlich bei Reaktionen in mehrphasigen Medien als Hilfsmittel zum Phasentransfer von Reaktanden dienen. Insbesondere seien Tetraalkyl- und Trialkyl-aralkyl-ammoniumsalze wie z.B. Tetrabutylammoniumbromid und Trimethyl-benzyl-ammoniumchlorid genannt. Verwendbare Alkalicyanide sind z.B. Natriumcyanid und Kaliumcyanid.

Die Reaktionstemperatur kann innerhalb eines größeren Bereichs variiert werden. Im allgemeinen arbeitet man zwischen 0 und 100°C, vorzugsweise zwischen 10 und 50°C.

Di Reaktionen werden im allgemeinen bei Normaldruck durchgeführt.

Zur Durchführung setzt man die Ausgangskomponenten im allgemeinen in äquimolaren Mengen ein. Ein Überschuß der einen oder anderen Komponente bringt keine wesentlichen Vorteile. Die Reaktionskomponenten werden im allgemeinen in einem oder mehreren der angegebenen Verdünnungsmittel zusammengegeben und zur Vervollständigung der Reaktion mehrere Stunden gerührt. Anschließend wird mit Toluol/Wasser geschüttelt, die organische Phase abgetrennt, mit

Wasser gewaschen und getrocknet. Nach Abdestillieren des Lösungsmittels im Vakuum fallen die neuen Verbindungen im allgemeinen in Form von Ölen an, die sich zum Teil nicht unzersetzt destillieren lassen. Sie können jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit und günstiger Warmblüteroxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Aqriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut,

ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

## Beispiel A

Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 6, 7.

Beispiel B

Test mit parasitierenden adulten Rinderzecken (Boophilus microplus res.)

Lösungsmittel: Alkylarylpolyglykoläther

Zur herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b. microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1.

Beispiel C

Test mit parasitierenden Fliegenlarven (Lucilia cuprina res.)

Emulgator: 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welches ca. 3 ml einer 20%igen Eigelbpulver-Suspension in Wasser enthält. Auf diese Eigelbpulver-Suspension werden 0,5 ml der Wirkstoffzuberteitung gebracht. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1,2.

Herstellungsbeispiele

Beispiel 1

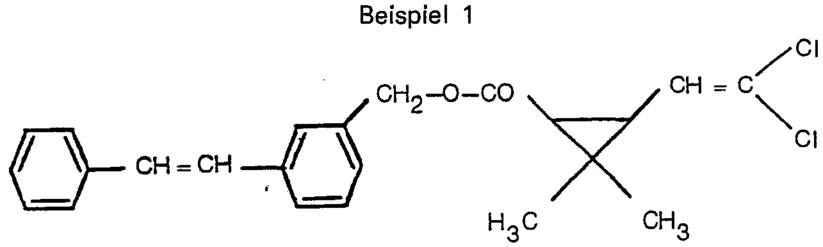

5 g (0,0238 Mol) 3-(2-Phenylvinyl)-benzylalkohol (ca. 90% cis-Isomeres) und 5,4 g (0.0238 Mol) 2,2-Dimethyl-3-dichlorvinyl-cyclopropancarbonsäurechlorid werden in 100 ml wasserfreiem Toluol gelöst und bei 20—25°C 3 g Pyridin, gelöst in 50 ml wasserfreiem Toluol, unter Rühren zugetropft. Anschließend wird weitere 3 Stunden bei 25°C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 5,6 g (58,7% der Theorie) 2,2-Dimethyl-3-dichlorvinyl-cyclopropancarbonsäure-3'-(2-phenylvinyl)-benzylester als gelbes Öl mit dem Brechungsindex $n_D^{24}$: 1,5853 (Isomerengemisch mit ca. 90% cis Stilben-Anteil).

Beispiel 2

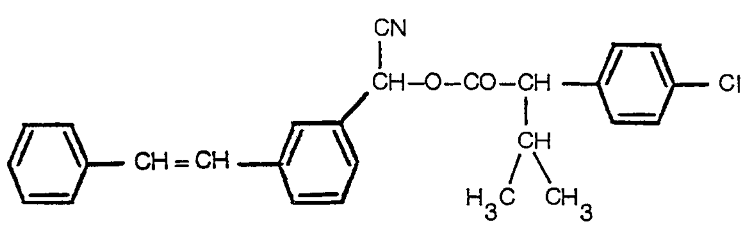

4,16 g (0,02 Mol) 3-(2-Phenyl-vinyl)benzaldehyd und 4,62 (0,02 Mol) α-Isopropyl-4-chlorphenylessigssäurechlorid werden zusammen unter Rühren bei 20—25°C zu einer Mischung von 1,5 g Natriumcyanid, 2 ml Wasser, 40 ml n-Hexan und 0,5 g Tetrabutylammoniumbromid getropft und dann 4

Stunden bei 20—25°C gerührt. Anschließend wird die Reaktionsmischung mit 300 ml Toluol versetzt und 2 mal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 6 g (69,9% der Theorie) $\alpha$-Isopropyl-4-chlorphenylessigsäure-3-(2-phenylvinyl)-$\alpha$-cyanobenzylester als gelbes Öl mit dem Brechungsindex $n_D^{22}$: 1,5879 (Isomerengemisch mit ca. 90% cis-Stilben-Anteil).

Analog Beispiel 1 oder 2 können die folgenden Verbindungen hergestellt werden (cis-Stilben-Anteil jeweils ca. 90%):

| Beispiel Nr: | Formel | Ausbeute (% der Theorie) | Brechungsindex: |
|---|---|---|---|
| 3 | 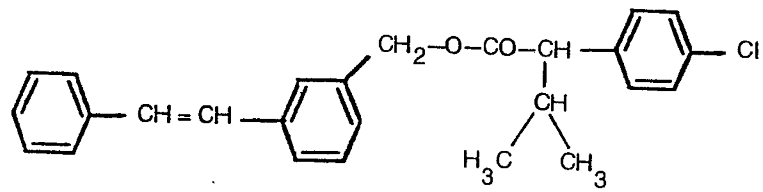 | 65,9 | $n_D^{22}$: 1,5911 |
| 4 | 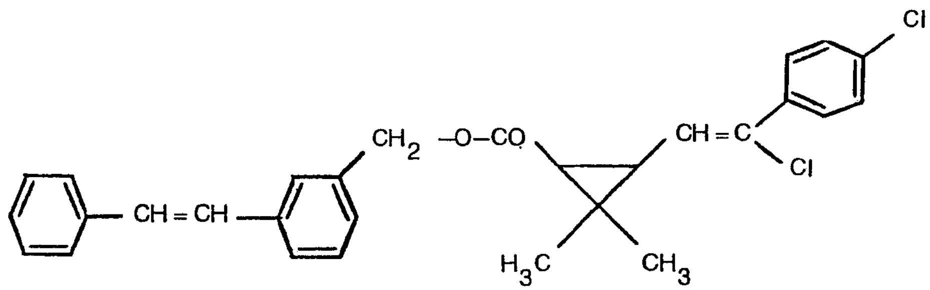 | 76,9 | $n_D^{22}$: 1,6049 |
| 5 | 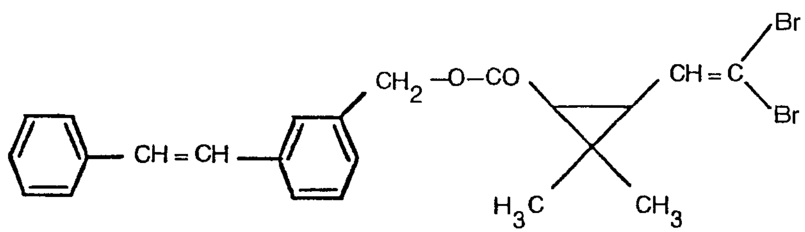 | 81,6 | $n_D^{22}$: 1,5849 |
| 6 | 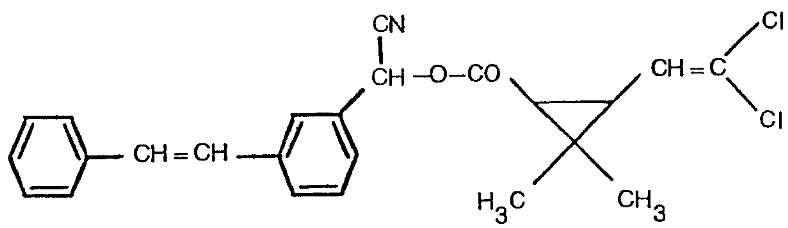 | 70,4 | $n_D^{22}$: 1,5846 |

| Beispiel Nr: | Formel | Ausbeute (% der Theorie) | Brechungsindex: |
|---|---|---|---|
| 7 | 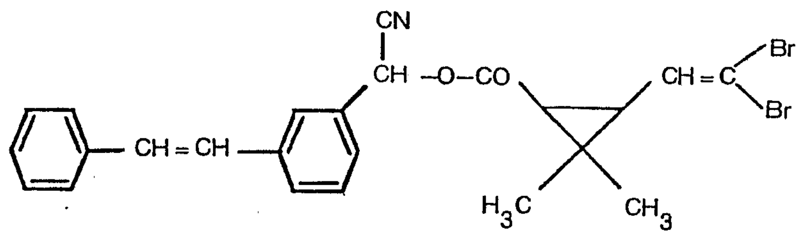 | 77,7 | $n_D^{22}$: 1,5907 |
| 8 | 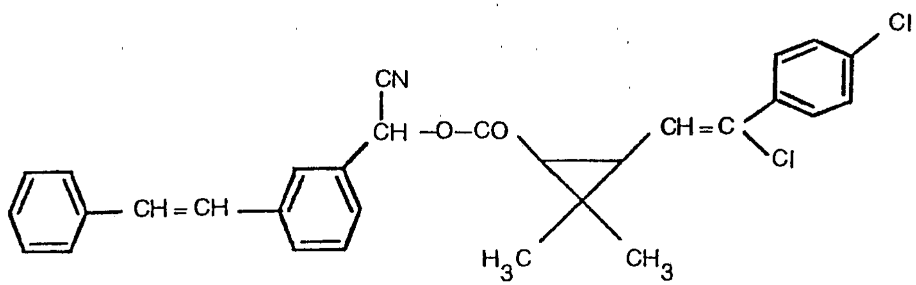 | 49,8 | |
| 9 | 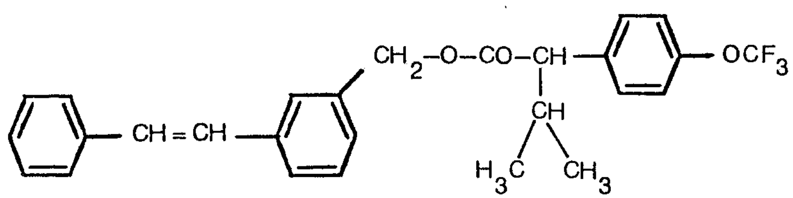 | 55,0 | $n_D^{23}$: 1,5424 |
| 10 | 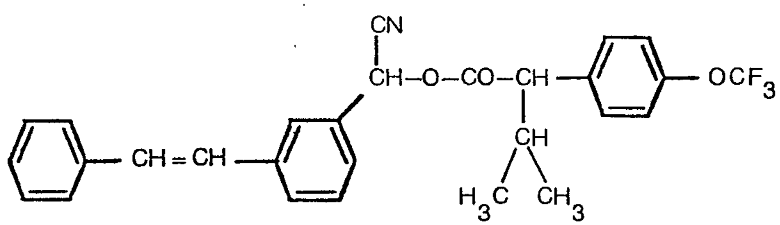 | | |

| Beispiel Nr: | Formel | Ausbeute (% der Theorie) | Brechungsindex: |
|---|---|---|---|
| 11 | 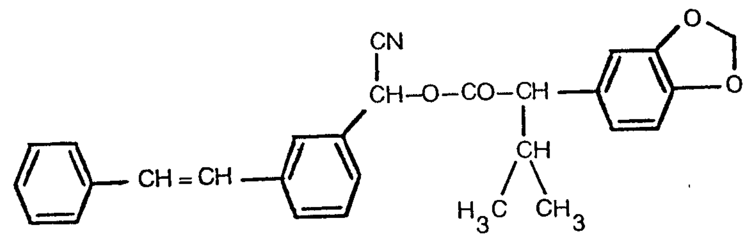 | | |
| 12 | 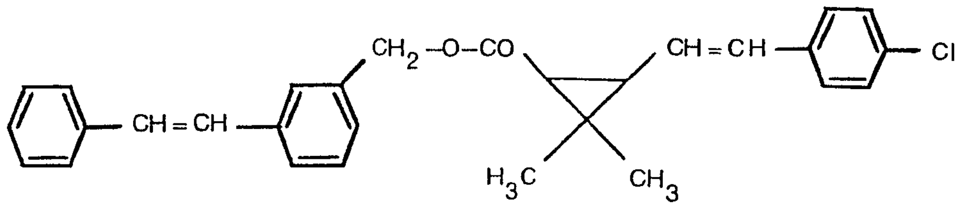 | | |
| 13 | 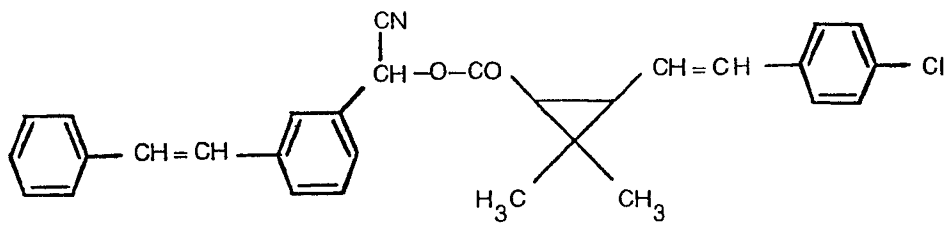 | 57,8 | |
| 14 | 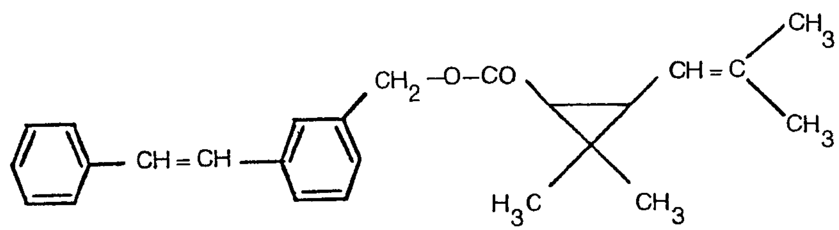 | 64,8 | $n_D^{22}$: 1,5812 |

| Beispiel Nr: | Formel | Ausbeute (% der Theorie) | Brechungs-index: |
|---|---|---|---|
| 15 | 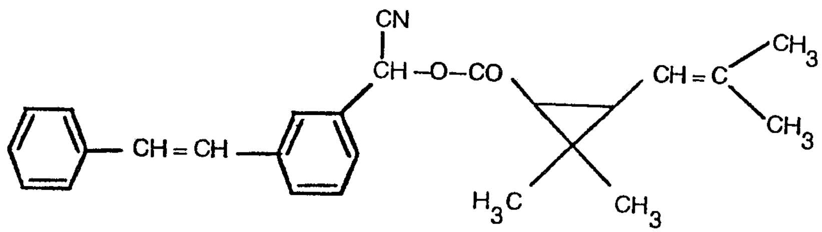 | | |
| 16 | 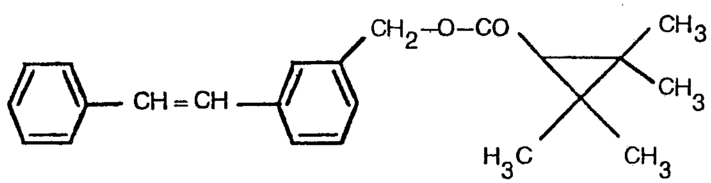 | | |
| 17 | 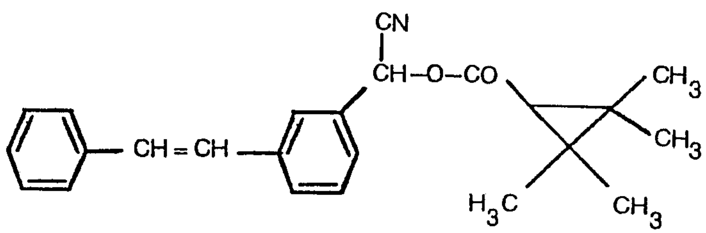 | | |
| 18 | 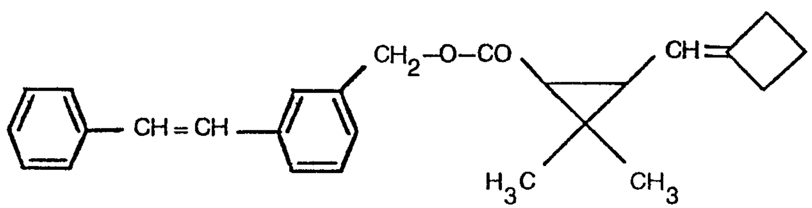 | | |

| Beispiel Nr: | Formel | Ausbeute (% der Theorie) | Brechungs-index: |
| --- | --- | --- | --- |
| 19 | 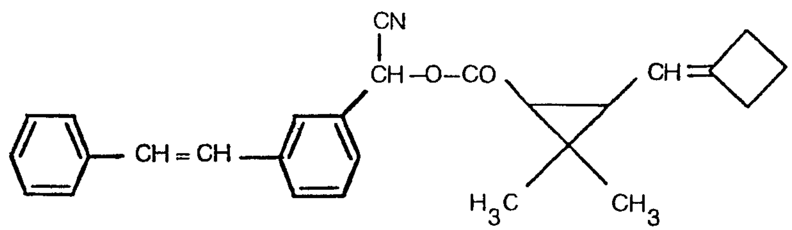 | | |
| 20 | 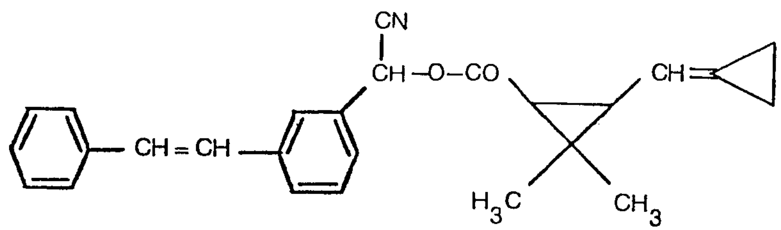 | | |
| 21 | 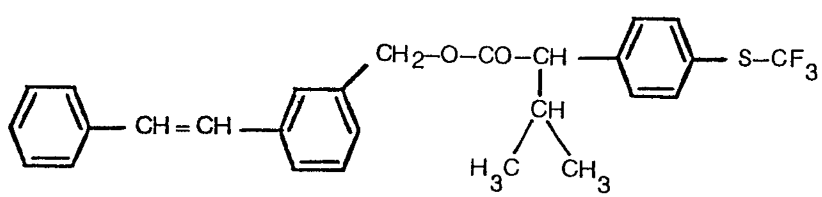 | 61,2 | $n_D^{23}$: 1,5662 |
| 22 | 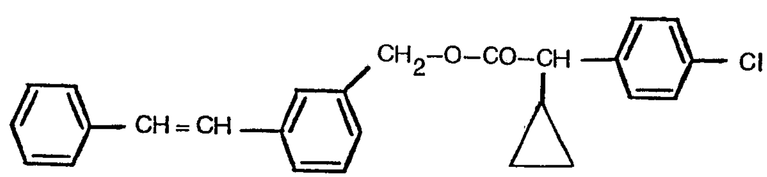 | | |

| Beispiel Nr: | Formel | Ausbeute (% der Theorie) | Brechungs-index: |
|---|---|---|---|
| 23 | 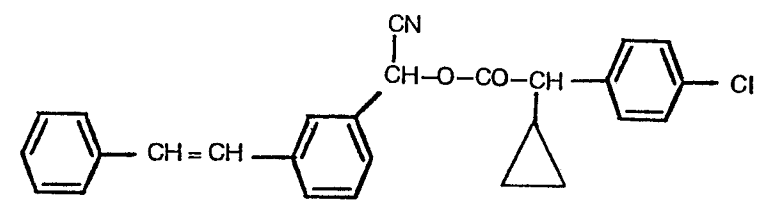 | 58,5 | |
| 24 | 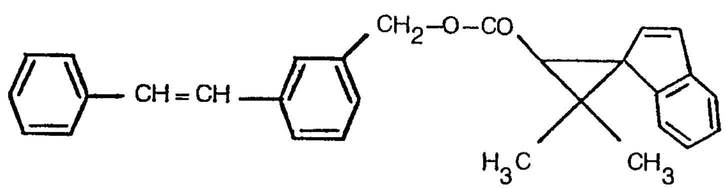 | | |
| 25 | 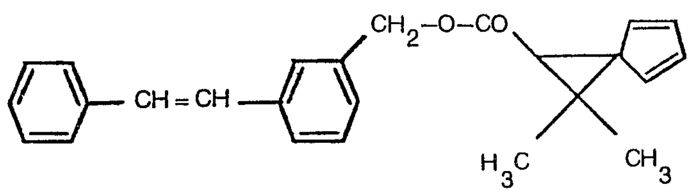 | | |

Unter Verwendung von 3-(2-Phenyl-vinyl)-benzylalkohol, welcher ca. 90% des trans-Isomeren enthält, können analog Beispiel 1 oder 2 die folgenden Verbindungen mit ca. 90% trans-Stilben-Anteil hergestellt werden:

| Beispiel Nr: | Formel | Ausbeute (% der Theorie) | Brechungs-index: |
|---|---|---|---|
| 26 | 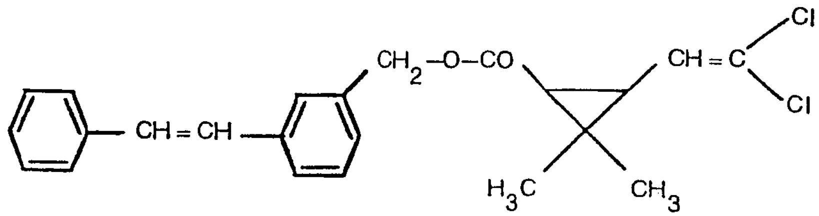 | 83,1 | $n_D^{22}$: 1,6058 |
| 27 | 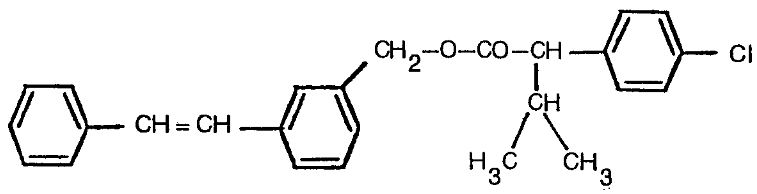 | 82,4 | $n_D^{22}$: 1,6119 |
| 28 | 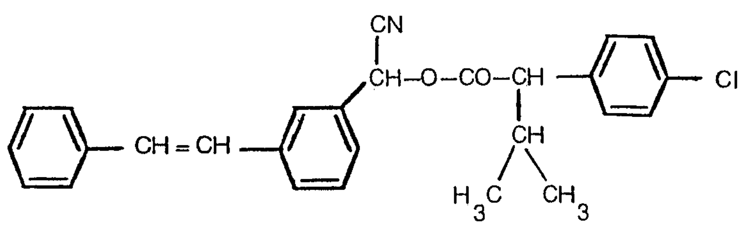 | | |
| 29 | 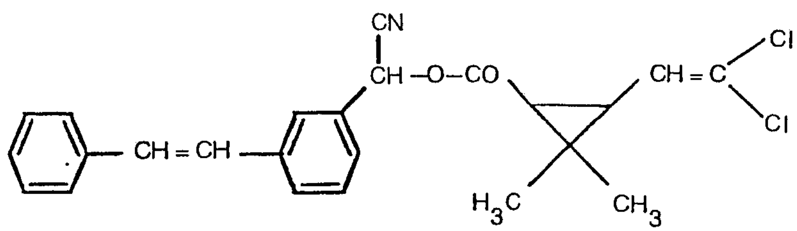 | 58,7 | |

Herstellung der Ausgangsverbindungen:

(a) cis-Stilben-derivate

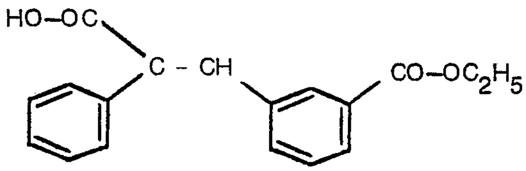

17,8 g (0,1 Mol) 3-Formyl-benzoesäureäthylester, 13,6 g (0,1 Mol) Phenylessigsäure und 10 g Triäthylamin werden in 30 ml Essigsäureanhydrid gelöst und 5 Stunden zum Rückfluß erhitzt. Nach dem Abkühlen wird das Reaktionsgemisch mit 300 ml Wasser versetzt und anschließend 20 Minuten zum Sieden erhitzt. Dann wird *heiß* vom festen Rückstand abdekantiert und dieser aus Methanol, das mit etwas Wasser versetzt wird, umkristallisiert. Man erhält 13 g (43,9% der Theorie) 3-(2-Carboxy-2-phenyl-vinyl)-benzoesäureäthylester als hellgelbe Kristalle vom Siedepunkt 139—140°C (Isomerengemisch mit ca. 90% cis-Anteil).

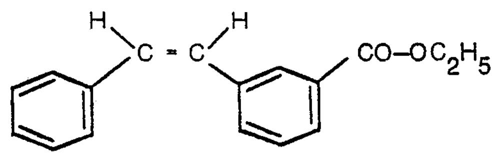

13 g (0,044 Mol) 3-(2-Carboxy-3-phenyl-vinyl)-benzoesäureäthylester werden in 100 ml Chinolin gelöst mit 1 g Kupferpulver versetzt und unter Rühren solange auf 220°C erhitzt, bis keine Kohlendioxidentwicklung mehr zu beobachten ist (ca. 1 Stunde). Anschließend wird sofort auf Raumtemperatur abgekühlt. Nach Zugabe von 300 ml Toluol zum Reaktionsgemisch wird 2 mal mit je 100 ml konzentrierter Salzsäure ausgeschüttelt, anschließend mit 500 ml Wasser gewaschen. Die organische Phase wird abgetrennt über Magnesiumsulfat getrocknet und das Lösungsmittel im Wasserstrahlvakuum abdestilliert, der Rückstand wird im Vakuum destilliert. Man erhält 10 g (90,2% der Theorie) 3-(2-Phenyl-vinyl)-benzoesäureäthylester als gelbes Öl mit dem Siedepunkt 165—170°C/2 Torr; (Isomerengemisch mit ca. 90% cis-Anteil).

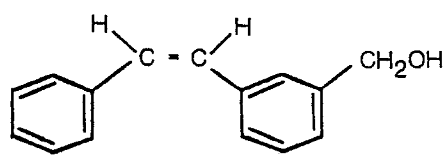

Zu 2,5 g Lithiumaluminiumhydrid in 50 ml wasserfreiem Tetrahydrofuran werden bei 25—30°C 10 g (0,03 Mol) 3-(2-Phenyl-vinyl)-benzoesäureäthylester, gelöst in 50 ml trockenem Tetrahydrofuran, unter gutem Rühren zugetropft. Anschließend wird 10 Stunden bei 22°C nachgerührt, der Reaktionsansatz dann auf 0°C abgekühlt und unter Rühren solange Eiswasser (60 ml) zugetropft, bis keine Wasserstoffentwicklung mehr zu beobachten ist. Der entstandene Niederschlag wird durch zutropfen von 30 ml konzentrierter Salzsäure gelöst und anschließend das Reaktionsgemisch 2 mal mit je 100 ml Toluol extrahiert. Die organische Phase wird abgetrennt, über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 5 g (61% der Theorie) 3-(2-Phenyl-vinyl)-benzylalkohol als gelbes Öl mit dem Brechungsindex $n_D^{22}$: 1,6286 (Isomerengemisch mit ca. 90% cis-Anteil).

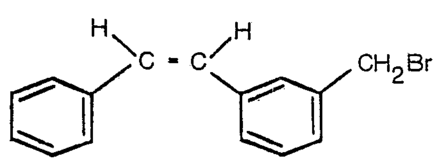

21 g (0,1 Mol) 3-(2-Phenyl-vinyl)-benzylalkohol werden in 200 ml wasserfreiem Toluol gelöst und bei 0—10°C unter Rühren 10 g Phosphortribromid zugetropft. Anschließend wird 2 Stunden bei 10°C und

noch 2 Stunden bei Raumtemperatur gerührt. Die Reaktionsmischung wird dann 2 mal mit je 500 ml Wasser ausgeschüttelt, die organische Phase abgetrennt, über Magnesiumsulfat getrocknet und das Lösungsmittel im Wasserstrahlvakuum abdestilliert. Letzte Lösungsmittelreste werden durch kurzes Andestillieren bei 60°C/2 Torr Badtemperatur entfernt. Man erhält 23 g (84,2% der Theorie) 3-(2-Phenyl-vinyl)-benzylbromid als gelbes Öl mit dem Brechungsindex $n_D^{22}$: 1,6376 (Isomerengemisch mit ca. 90% cis-Anteil).

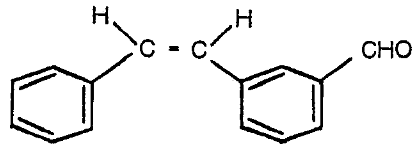

47 g (0,172 Mol) 3-(2-Phenyl-vinyl)-benzylbromid und 60 g Hexamethylentetramin in 400 ml Methylenchlorid werden 3 Stunden am Rückfluß erhitzt, anschließend wird auf 5—10°C abgekühlt und der entstandene Niederschlag abgesaugt. Dieser wird mit 100 ml Methylenchlorid gewaschen, trocken gesaugt und dann in 600 ml 50%iger wässriger Essigsäure 3 Stunden am Rückfluß erhitzt. Danach wird auf Raumtemperatur abgekühlt und 45 ml konzentrierte Salzsäure hinzugefügt. Das Reaktionsgemisch wird dann mit 800 ml Wasser versetzt und zweimal mit je 200 ml Methylenchlorid extrahiert, die organische Phase anschließend abgetrennt und über Natriumsulfat getrocknet. Das Lösungsmittel wird im Wasserstrahlvakuum abdestilliert, der ölige Rückstand wird anschließend destilliert. Man erhält 15 g (41,9% der Theorie) 3-(2-Phenyl-vinyl)-benzaldehyd als farblose Flüssigkeit mit dem Siedepunkt 145—150°C/2 Torr. (Isomerengemisch mit einem ca. 90%igen cis-Anteil).

(b) trans-Stilben-derivate

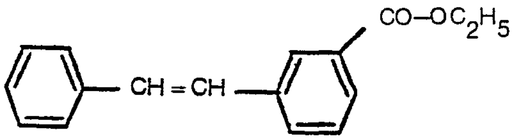

Zu einer Mischung von 300 ml Tetrahydrofuran und 46,4 g (0,22 Mol) einer methanolischen Natriummethylatlösung werden bei 0—5°C unter Rühren 45,6 g (0,2 Mol) Benzylphosphonsäurediäthylester, gelöst in 50 ml Tetrahydrofuran, zugetropft. Man läßt 2 Stunden bei 0°C nachrühren und tropft dann, ebenfalls bei 0°C und unter Rühren, 35,6 g (0,2 Mol) 3-Formylbenzosäureäthylester, gelöst in 50 ml Tetrahydrofuran, zu. Man läßt dann 12 Stunden bei 20—25°C rühren, versetzt anschließend das Reaktionsgemisch mit 300 ml Toluol und schüttelt dann 2 mal mit je 600 ml Wasser aus. Die organische Phase wird abgetrennt, über Magnesiumsulfat getrocknet und das Lösungsmittel im Wasserstrahlvakuum abdestilliert. Der ölige Rückstand wird bei 160—190°C und einem Druck von 3 Torr destilliert. Das so erhaltene halbkristalline Produkt wird in wenig Petroläther gelöst und auf 0°C abgekühlt. Die ausgefallenen Kristalle werden abgesaugt und getrocknet. Man erhält 19 g (37,7% der Theorie) 3-(2-Phenyl-vinyl)-benzoesäureäthylester als farblose Kristalle mit dem Schmelzpunkt 82°C (Isomerengemisch ca. 90% trans-Konfiguration).

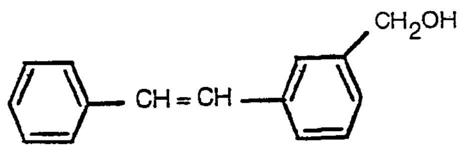

Nach dem oben beschriebenen Verfahren (siehe "cis-Stilben-derivate") läßt sich 3-(2-Phenyl-vinyl)-benzoesäureäthylester (mit 90%igem trans-Anteil) mit Lithiumaluminiumhydrid zu 3-(2-Phenyl-vinyl)-benzylalkohol (Isomerengemisch ca. 90% trans-Konfiguration) reduzieren.

Ausbeute: 13,5 g (85,7% der Theorie), Schmelzpunkt 84°C, farblose Kristalle.

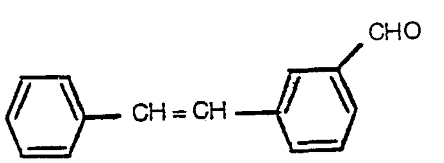

Analog dem oben beschriebenen Herstellungsweg (siehe "cis-Stilben-derivate") erhält man ausgehend vom 3-(2-Phenyl-vinyl)-benzylalkohol (ca. 90% trans-Anteil) 3-(2-Phenyl-vinyl)-benzaldehyd in einer Ausbeute von 82,4% der Theorie als farblose Kristalle mit dem Schmelzpunkt 90°C (Isomerengemisch mit ca. 90%igem trans-Anteil).

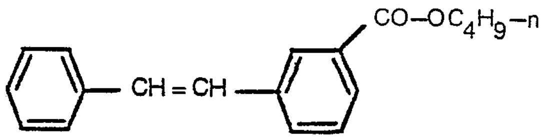

2,3 g (0,1 Mol) Natrium werden in 50 ml Äthanol aufgelöst und diese Lösung wird tropfenweise zu einer Lösung von 38,9 g (0,1 Mol) Benzyl-triphenyl-phosphoniumchlorid in 150 ml Äthanol bei einer Innentemperatur von —5 bis 0°C gegeben. Dann gibt man dazu 20,6 g (0,1 Mol) 3-Formylbenzoesäure-n-butylester, hält unter Rühren die Mischung zwei Stunden bei 0 bis 5°C und läßt langsam auf Raumtemperatur kommen. Nach Einengen wird mit Toluol extrahiert, filtriert und das Lösungsmittel im Vakuum abdestilliert. Man erhält 21,3 g (75% der Theorie) 3-(2-Phenyl-vinyl)-benzoesäure-n-butylester als leicht gelbliches Öl (cis/trans-Isomerengemisch im Verhältnis 1:1).

**Patentansprüche**

1. Stilbenderivate der Formel

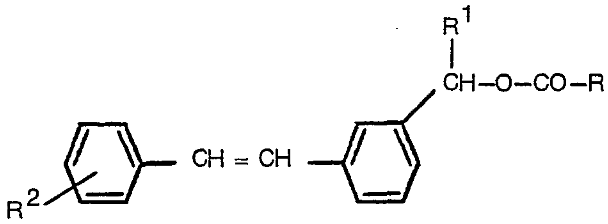

in welcher

R für einen offenkettigen oder cyclischen Alkylrest steht, der gegebenenfalls substituiert ist durch Halogen, Alkyl, Cycloalkyl, Alkenyl, welches gegebenenfalls durch Halogen substituiert ist, Styryl, welches gegebenenfalls durch Halogen substituiert ist, Phenyl, welches gegebenenfalls durch Halogen, Alkyl, Alkoxy, Alkylthio, Halogenalkyl, Halogenalkoxy, Halogenalkylthio oder Methylendioxy substituiert ist, spirocyclisch verknüpftes Cycloalkenyl, welches gegebenenfalls benzannelliert ist und

$R^1$ für Wasserstoff, Cyan oder Alkinyl steht und

$R^2$ für Wasserstoff, Halogen oder Alkyl steht.

2. Verfahren zur Herstellung der neuen Stilbenderivate der Formel

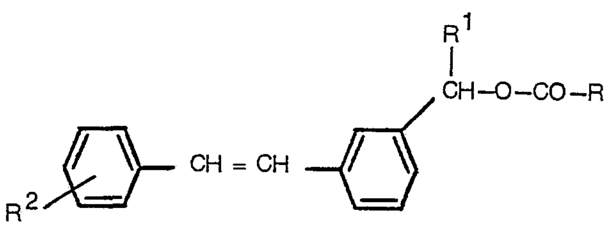

in welcher

R für einen offenkettigen oder cyclischen Alkylrest steht, der gegebenenfalls substituiert ist durch Halogen, Alkyl, Cycloalkyl, Alkenyl, welches gegebenenfalls durch Halogen substituiert ist, Styryl, welches gegebenenfalls durch Halogen substituiert ist, Phenyl, welches gegebenenfalls durch Halogen, Alkyl, Alkoxy, Alkylthio, Halogenalkyl, Halogenalkoxy, Halogenalkylthio oder Methylendioxy substituiert ist, spirocyclisch verknüpftes Cycloalkenyl, welches gegebenenfalls benzannelliert ist und

$R^1$ für Wasserstoff, Cyan oder Alkinyl steht und

$R^2$ für Wasserstoff, Halogen oder Alkyl steht,

dadurch gekennzeichnet, daß man Carbonsäuren der Formel II

$$R—COOH \qquad (II)$$

in welcher

R die oben angegebene Bedeutung hat,
oder ein Salz oder Säurechlorid derselben mit Alkoholen der Formel III

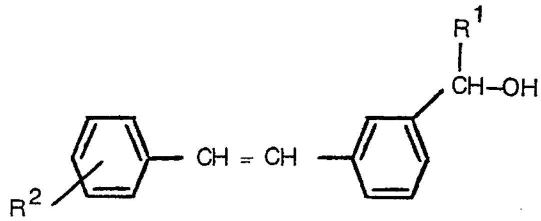

(III)

in welcher

die Reste $R^1$ und $R^2$ die oben angegebene Bedeutung haben,
oder für den Fall, daß $R^1$ für CN steht, die Säurechloride der Säuren der Formel II mit den den Alkoholen der Formel III zugrundeliegenden Aldehyden und Alkalicyaniden, gegebenenfalls in Gegenwart eines Säurebinders und gegebenenfalls in Gegenwart eines Verdünnungsmittels umsetzt.

3. Alkohole der Formel III

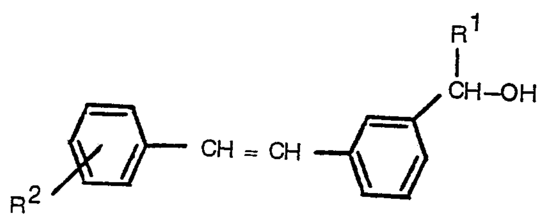

(III)

in welcher

$R^1$ für Cyan steht und

$R^2$ für Wasserstoff, Halogen oder Alkyl steht.

4. Insektizide Mittel gekennzeichnet durch einen Gehalt an mindestens einem Stilbenderivat gemäß Anspruch 1.

5. Verwendung von Stilbenderivaten gemäß Anspruch 1 zur Bekämpfung von Insekten.

**Revendications**

1. Dérivés de stilbène de formule

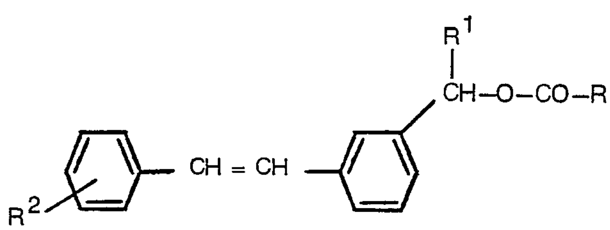

dans laquelle

R représente un reste alkyle à chaîne ouverte ou cyclique, qui est éventuellement substitué par halogène ou par alkyle, cycloalkyle, alcényle, qui est éventuellement substitué par halogène, styryle, qui est éventuellement substitué par halogène, phényle, qui est éventuellement substitué par halogène ou par alkyle, alcoxy, alkylthio, halogénoalkyle, halogénoalcoxy, halogénoalkylthio, méthylènedioxy ou un reste cycloalcényle relié sous forme d'un composé spirocyclique et qui est éventuellement accolé à un noyau benzo, et

$R^1$ représente l'hydrogène ou un groupe cyano ou alcynyle et

$R^2$ représente l'hydrogène ou un halogène ou un groupe alkyle.

2. Procédé pour la fabrication des nouveaux dérivés de stilbène de formule

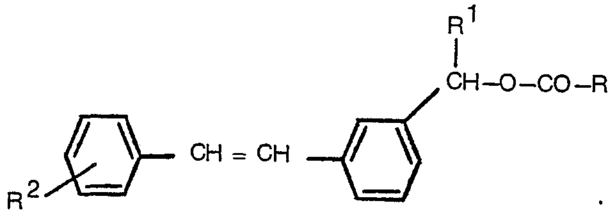

(I)

dans laquelle

R représente un reste alkyle à chaîne ouverte ou cyclique, qui est éventuellement substitué par halogène ou par alkyle, cycloalkyle, alcényle, qui est éventuellement substitué par halogène, styryle, qui est éventuellement substitué par halogène, phényle, qui est éventuellement substitué par halogène ou par alkyle, alcoxy, alkylthio, halogénoalkyle, halogénoalcoxy, halogénoalkylthio méthylènedioxy ou un reste cycloalcényle relié sous forme d'un composé spirocyclique et qui est éventuellement accolé à un noyau benzo, et

$R^1$ représente l'hydrogène ou un groupe cyano ou alcynyle et

$R^2$ représente l'hydrogène ou un halogène ou un groupe alkyle, caractérisé en ce que l'on fait réagir des acides carboxyliques de formule II

$$R—COOH \qquad (II)$$

dans laquelle

R a la signification indiquée ci-dessus,

ou un de leurs sels ou de leurs chlorures d'acides avec des alcools dé formule

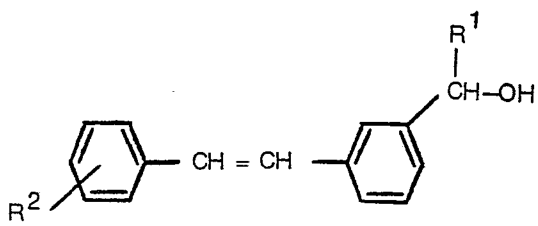

(III)

dans laquelle

les restes $R^1$ et $R^2$ ont la signification indiquée ci-dessus, ou bien, dans le cas où $R^1$ représente le reste CN, on fait réagir les chlorures d'acides des acides de formule II avec les aldéhydes dont dérivent les alcools de formule III et des cyanures alcalins, éventuellement en présence d'un accepteur d'acide et éventuellement en présence d'un agent diluant.

3. Alcools de formule

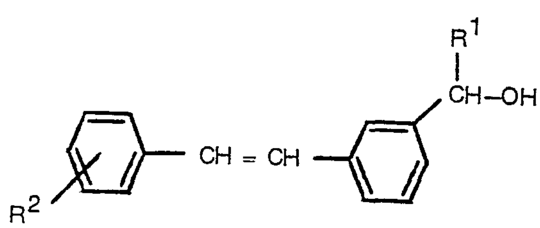

(III)

dans laquelle

$R^1$ est un groupe CN et

$R^2$ représente un atome d'hydrogène ou d'halogène ou un groupe alkyle.

4. Agents insecticides, caractérisés en ce qu'ils contiennent au moins un dérive de stilbène selon la revendication 1.

5. Utilisatiǒn des dérivés de stilbène selon la revendication 1 pour la lutte contre les insectes.

**Claims**

1. Stilbene derivatives of the formula

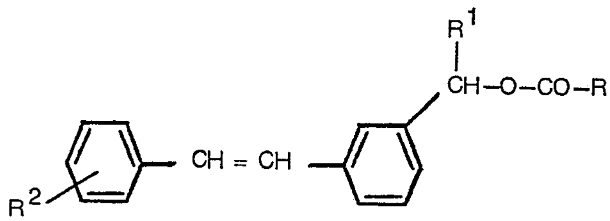

(I)

in which

R represents an open-chain or cyclic alkyl radical which is optionally substituted by halogen, alkyl, cycloalkyl, alkenyl, which is optionally substituted by halogen, styryl, which is optionally substituted by halogen, phenyl, which is optionally substituted by halogen, alkyl, alkoxy, alkylthio, halogenoalkyl, halogenoalkoxy, halogenoalkylthio or methylenedioxy, or cycloalkenyl which is linked in a sprirocyclic manner and is optionally benzo-fused,

$R^1$ represents hydrogen, cyano or alkinyl and

$R^2$ represents hydrogen, halogen or alkyl.

2. Process for the preparation of the new stilbene derivatives of the formula

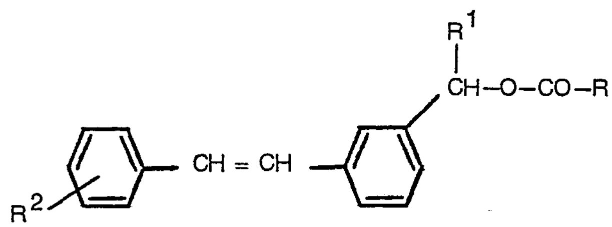

(I)

in which

R represents an open-chain or cyclic alkyl radical which is optionally substituted by halogen, alkyl, cycloalkyl, alkenyl, which is optionally substituted by halogen, styryl, which is optionally substituted by halogen, phenyl, which is optionally substituted by halogen, alkyl, alkoxy, alkylthio, halogenoalkyl, halogenoalkoxy, halogenoalkylthio or methylenedioxy, or cycloalkenyl which is linked in a spirocyclic manner and is optionally benzo-fused,

$R^1$ represents hydrogen, cyano or alkinyl and

$R^2$ represents hydrogen, halogen or alkyl, characterised in that carboxylic acids of the formula II

$$R—COOH \qquad (II)$$

in which

R has the meaning indicated above,

or a salt or acid chloride thereof, are reacted with alcohols of the formula III

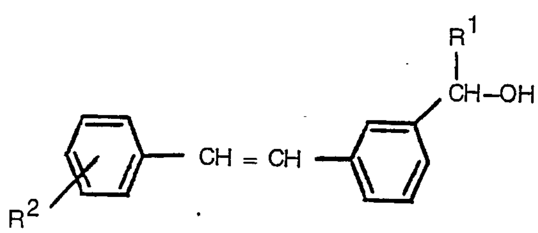

(III)

in which

the radicals $R^1$ and $R^2$ have the meaning indicated above,

or in the case where $R^1$ represents CN the acid chlorides of the acids of the formula II are reacted with the aldehydes and alkali metal cyanides from which the alcohols of the formula III are derived, if appropriate in the presence of an acid binder and if appropriate in the presence of a diluent.

3. Alcohols of the formula III

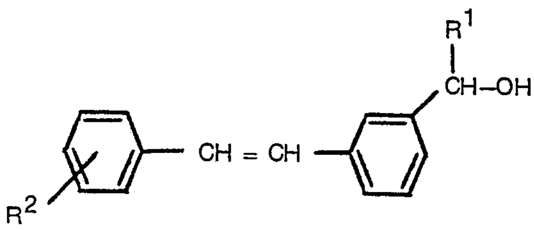

(III)

in which

$R^1$ represents cyano and $R^2$ represents hydrogen, halogen or alkyl.

4. Insecticidal agents, characterised in that they contain at least one stilbene derivate according to Claim 1.

5. Use of stilbene derivatives according to Claim 1 for combating insects.